# EUROPEAN PATENT SPECIFICATION

(11) **EP 3 815 683 B1**
(45) Date of publication and mention of the grant of the patent: **16.07.2025**
(21) Application number: 20020497.2
(22) Date of filing: 29.10.2020
(51) Int. Cl.: A61K 31/198, A61K 45/00, A61K 33/30, A61P 17/14, A61K 31/191, A61K 31/375

(54) **TREATMENT OF ANDROGENETIC ALOPECIA WITH A COMBINATION COMPRISING ZINC, ARGININE GLUCONATE AND ASCORBIC ACID**
BEHANDLUNG VON ANDROGENETISCHER ALOPEZIE MIT EINER KOMBINATION AUS ZINK, ARGININ-GLUCONAT UND ASCORBINSÄURE
TRAITEMENT DE L'ALOPÉCIE ANDROGÉNÉTIQUE PAR UNE COMBINAISON COMPRENANT DU ZINC, DU GLUCONATE D'ARGININE ET DE L'ACIDE ASCORBIQUE

(30) Priority: 31.10.2019 IT 201900020118
(43) Date of publication of application: 05.05.2021
(73) Proprietor: Biogena S.r.l., 20129 Milano (IT)
(72) Inventor: BORTOLIN, Vittorino, 24030 Presezzo (BG) (IT)
(74) Representative: Cattaneo, Elisabetta

(56) References cited:
- CN-A- 106 937 957
- US-A1- 2008 051 351
- DATABASE GNPD [online] MINTEL; 18 September 2018 (2018-09-18), ANONYMOUS: "Wash Restorative Softening Shampoo", XP055726337, retrieved from www.gnpd.com Database accession no. 5983829

## Description

The present invention relates, in general, to a formulation or remedy for androgenetic alopecia.

In particular, the present invention relates to such a remedy, consisting of a combination of substances or formulation.

As well as performing a protective function, hair also has an important aesthetic value, greatly contributing to the external image and self-esteem of an individual.

It is well known that many people, especially women, place considerable importance on their hair, taking care to choose the right hairstyle, care for and treat their hair, often, with considerable effort.

Hair loss is, for both sexes, an event with strong psychological repercussions, sometimes a real trauma: it is well known that the loss of hair that occurs, for example, during chemotherapy treatment is a problem that requires the use of wigs to limit the negative perception of one's condition.

Among the many causes of hair loss, the most common is by far the one involving male steroid hormones: this form of hair loss is known by the scientific name of androgenetic alopecia.

In androgenetic alopecia there is a thinning of the hair, which may be more or less marked and at a varying rate, which in men is located in the frontal-temporal area and/or tonsure area, while in women it is almost always distributed over the entire upper part of the head. The phenomenon manifests itself in men much more than in women, who generally suffer from it at an older age, usually after menopause.

Androgenetic alopecia begins with a progressive miniaturization of the hair follicle and ends with its complete atrophy. This involution of the hair follicle is caused by the presence of a substance, DHT (di-hydro-testosterone), a hormonal derivative of testosterone, which has the ability to bind to specific receptors of the hair follicle, causing its progressive deterioration.

However, there is a different susceptibility of hair follicles to the action of dihydrotestosterone, greater in some areas of the scalp than in others, greater in some individuals than in others. The increased individual susceptibility of hair follicles to dihydrotestosterone is genetic: a family predisposition to androgenetic alopecia has been established.

Dihydrotestosterone is universally recognized as the hormone responsible for the progressive miniaturization of the hair follicle, and therefore for androgenetic alopecia, while testosterone as such is completely inactive in this respect.

The transformation of testosterone into di-hydro-testosterone occurs locally, in the hair follicle itself and in the attached sebaceous gland, through the intervention of a specific enzyme called 5-alpha reductase type II.

Several formulations have been tested in the past to locally inhibit the enzyme 5-alpha reductase type II, achieving some success in preventing baldness. So far, the active ingredient that has proved most effective in this respect is Finasteride, a drug used mainly systemically for the treatment of prostate cancer.

This active ingredient, although generally well tolerated by the body, nevertheless has some non-negligible side effects, such as erectile dysfunction, gynecomastia, memory loss, chronic asthenia, loss of sexual desire, muscle hypotrophy, dry skin, slowing of cognitive processes, depression, anxiety, insomnia, immune depression and others. While these drawbacks may be accepted with a certain serenity in the life-saving treatment of a tumour, it is clear that the same cannot be said for the mere improvement of a purely aesthetic condition.

It would therefore be desirable to have active ingredients able to combat androgenetic alopecia by acting locally, without inducing major side effects.

The aim of the invention is to propose a composition of substances that overcomes the aforementioned drawbacks and that makes it possible to eliminate, or at least substantially reduce, hair loss due to androgenetic causes, without presenting the same drawbacks as Finasteride and proving, if possible, just as effective. Such purpose is achieved by a formulation against androgenetic alopecia characterised in that it comprises zinc and arginine gluconates combined with ascorbic acid, the last acting as initiator.

It should be noted that zinc and arginine gluconates have already been attributed a certain inhibitory activity against the enzyme 5-alpha reductase in the past, however not comparable with that performed by Finasteride.

"Wash Restorative Softening Shampoo", XP055726337 from www.gnpd.com Database accession no. 5983829 discloses a shampoo formulation comprising zinc gluconate, arginine and ascorbic acid but not arginine gluconate.

CN 106 937 957 A discloses a treatment of androgenetic alopecia via topical compositions comprising (inter alia) vitamin C. US2008/051351 A1 discloses a treatment of alopecia such as androgenetic alopecia and a hair gel formulation comprising zinc gluconate.

The present invention, consisting of the combination of zinc and arginine gluconates with ascorbic acid, allows important advantages. In fact, this combination makes it possible to simultaneously achieve two objectives of particular importance in the fight against androgenetic alopecia, precisely:
1. the inhibition of the 5-alpha reductase type II enzyme at the hair follicle level, with effectiveness comparable, or even higher, than Finasteride;
2. increased gene expression of the SOX9 protein by hair follicle cells. This particular effect, completely unexpected and independent of the former, can lead (according to available scientific data) to a stimulation of the dermal papilla of the hair follicle and to a further contrasting action of hair miniaturization, typical of androgenetic alopecia.

The attached figures illustrate the results of the in-vitro assays performed which confirm the above;

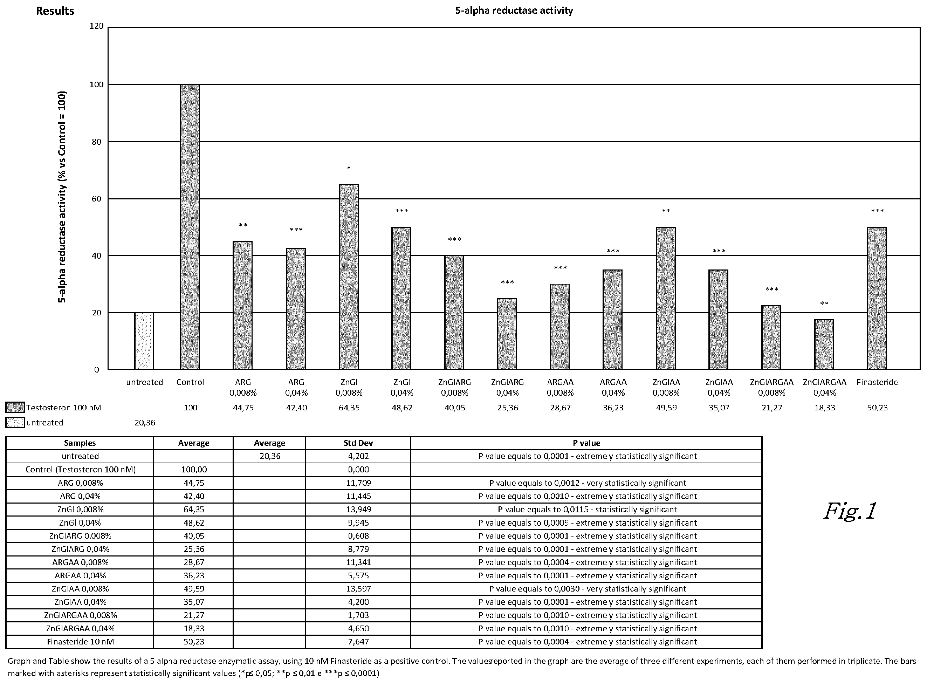

1) figure 1 shows the results of an enzyme assay on 5-alpha reductase, using Finasteride 100 nM as positive control, the values shown in the graph being the average of three different experiments, each performed in triplicate and the bars marked with asterisks representing statistically significant values ^{(*p≤}0.05; ^{**p}≤ 0.01 and ^{***p}≤ 0.0001);

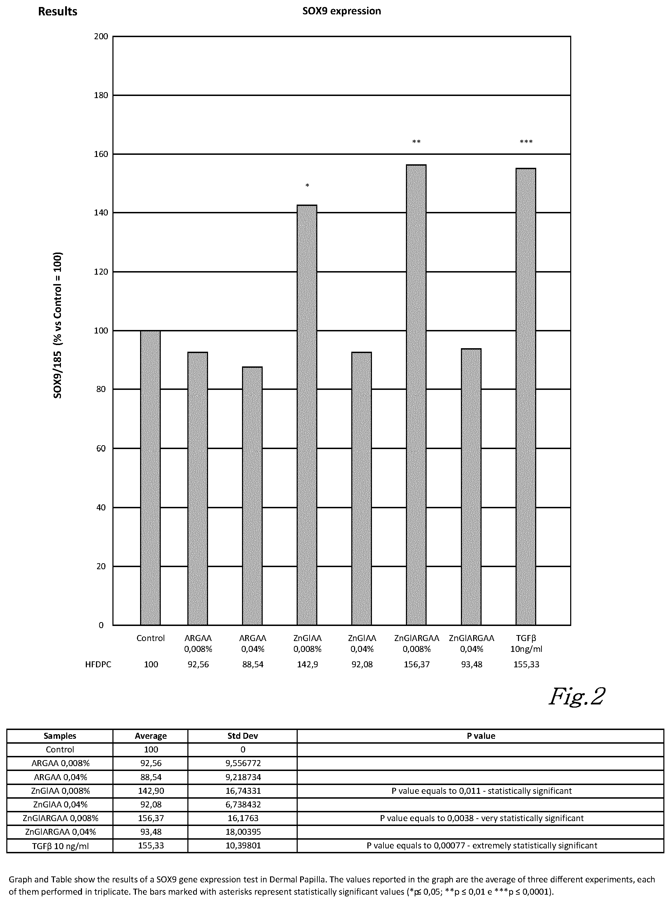

2) figure 2 shows the results of an expression test of the SOX9 gene in the dermal papilla. The values reported are the average of three different experiments, each of them performed in triplicate and the bars marked with asterisks representing statistically significant values ^{(*p≤}0,05; ^{**p}≤ 0,01 and ^{***p}≤ 0,0001). In this case, the Transformer Growth Factor beta type (TGF β)was used as the reference standard.

Below is a description of how the two tests mentioned above were performed:
Table 1 below shows the substances tested.

ZnGl stands for zinc gluconate, AA stands for ascorbic acid and ARG stands for arginine gluconate.

**Table 1:**

| Sample no. | Compounds | Physical state |
|---|---|---|
| 1 | ARG | liquid |
| 2 | ZnGl | liquid |
| 3 | ZnGlARG | liquid |
| 4 | ARGAA | liquid + powder |
| 5 | ZnGlAA | liquid + powder |
| 6 | ZnGlARGAA | liquid + powder |

### 5-ALPHA REDUCTASE TYPE II ENZYME ASSAY

8x10³ dermal papilla cells of the human follicle are seeded in 96 well plates and treated with the compounds to be examined and with 100 nM testosterone for 24 hours.

Coverage of 20 ng/ml of BSA-DHT at 4 °C in 100 µl of 50 mM sodium carbonate, pH 9.0. is performed.

After washing in PBS, the plate containing BSA-DHT is incubated with 50 µl of cell supernatant, to which 50 µl of biotin-conjugated primary anti-DHT antibody is added, dissolved in PBS containing 1% by weight of BSA.

The plate is washed three times in PBS two hours later and incubated with 100 µl of streptavidin-HRP 5 µg/ml in PBS containing 1% by weight of BSA.

The amount of DHT is measured by colorimetric reaction, using a solution of 0.5 mg/ml OPD at 0.012% by weight.

The plate is incubated at room temperature until colour development and the absorbance of the sample is measured at 490 nm with a Perkin Elmer Victor3 plate reader.

The results are shown in Fig. 1.

These results show that the ZnGl-ARG-AA combination, which is the subject of this invention, gives the best results, superior to those of Finasteride itself, used as a reference standard.

### ASSAY ON THE EXPRESSION OF THE SOX9 GENE IN THE DERMAL PAPILLA

To analyse gene expression in basal conditions,10⁶ dermal papilla cells from the human follicle are sown in 6 well plates, incubated for 24 hours with test compounds, and then processed for total RNA extraction. Total RNA is extracted with a GenElute Mammalian Total RNA Purification Kit (Sigma), according to the manufacturer's instructions. Everything is treated with DNase I at 37 °C for 30 minutes to eliminate any contaminating genomic DNA.

The first strand cDNA is synthesized from 0.5 - 1 µg, using the RevertAid First Strand cDNA Synthesis Kit (Fermentas). RT-PCR is performed at room temperature (TA), using gene specific primers and the QuantumRNA 18S internal standard (Ambion) according to manufacturer's instructions. The QuantumRNA kit contains primers to amplify 18S rRNA along with competimers that reduce the amplified 18S rRNA product within the range to allow it to be used as endogenous standard.

The amplification reactions are performed with the following general scheme: 2 min at 94°C followed by 35 cycles of 94°C for 30s, annealing temperature (specific for each gene) for 30s, and 72°C for 30-60s, with a 10 min final extension at 72°C.

The PCR products obtained are loaded on 1.5% agarose gel, and the amplification bands are visualized and quantified with the Geliance 200 Imaging system (Perkin Elmer). The amplification band corresponding to the gene analysed is normalized to the amplification band corresponding to the 18S. The values obtained are finally converted into percentage values by considering the measure of the untreated controls as 100%.

The results are shown in Fig. 2.

These results show that the ZnGl-ARG-AA combination, which is the subject of this invention, gives the best results, comparable to those obtained with TGF β used as a reference standard.

The present invention, as we have just seen, consists of a formulation containing zinc and arginine gluconates combined with ascorbic acid, to be used against androgenetic alopecia, acting primarily by inhibiting the enzyme 5-alpha reductase type II, and secondarily by inducing the SOX9 protein.

The formulation according to the present invention consists of two separate phases, to be mixed at the moment of use, this being a fundamental requirement to guarantee the stability over time of the components and therefore their respective efficacy: the first phase can be liquid or creamy or in the form of gel and contains zinc and arginine gluconates and relative adjuvants and excipients, while the second phase can be in the form of powder or suspension of solid particles in an oily or otherwise anhydrous vehicle and contains ascorbic acid and relative adjuvants and excipients.

The final preparation resulting from the union of the two aforementioned phases can be used for topical applications on the scalp according to different methods, such as massages, brushing, subcutaneous injections, sprays, shampoos or even through technologies and equipment of all kinds, the latter typically chosen in the group including equipment for skin stimulation with electromagnetic pulses, equipment for phototherapy using laser light.

The term "relative adjuvants and excipients" of zinc gluconates and arginine or ascorbic acid according to the present invention means at least one compound chosen from the group comprising Niacinamide Ascorbate, Ascorbyl Retinoate, Vanillyl Butyl Ether, Menthol, Anethole, Ellagic Acid, Panthenol, Tocopherol, Tocopheryl Acetate, Citric Acid, Lipoic Acid, Lecithin, Magnesium Ascorbate, Magnesium Ascorbyl Phosphate, Sodium Ascorbate, Sodium Ascorbyl Phosphate, Calcium Ascorbate, Ascorbyl Tetraisopalmitate, 3-O Ethyl Ascorbic Acid, Ascorbyl Glucoside, Glyceryl Octyl Ascorbic Acid, Tetrahexyldecyl Ascorbate.

According to a particularly advantageous embodiment of the present invention, it is expected that arginine gluconate, an essential component of the present invention, can be obtained during production starting from arginine base, salifying the same with stoichiometric quantities of gluconic acid or the relative precursor gluconolactone.

The formulation may comprise the further addition of arginine in the form of a free, non-salified base.

It is also possible that the formulation according to the present invention contains additional amounts of gluconic acid or gluconolactone.

Advantageously, the formulation envisages that the final preparation intended for application is obtained at the time of use or at a time very close to such use, since the formulation is not very stable and tends to degrade after about four days. Advantageously, the formulation according to the present invention is sold as a kit, consisting of two separate phases, packaged in two different compartments, to be mixed just before use, each of which contains some components of the overall formula to the exclusion of others and vice versa.

The formulation according to the present invention may include other ingredients such as excipients, preservatives, stabilizers, solvents, charges, fillers, and the like typically chosen from the group comprising Ethyl Alcohol, Isopropyl Alcohol, Propylene Glycol, Butylene Glycol, Glycerin, Polysorbate 20, PEG-40 Hydrogenated Castor Oil, Laureth-9, Dimethicone, Silica, Magnesium Stearate, Piroctone Olamine, Cyclopirox Olamine.

It may also contain synergistic adjuvants or compounds, known in themselves, typically chosen from the group comprising Soy Isoflavones, Genistein, Serenoa Serrulata Fruit Extract, Minoxidil. Without being bound by theory, the formulation of the present invention has an effect on 5-alpha reductase type II, acting like Finasteride, but without presenting the same undesirable side effects. It also has the ability to increase the gene expression of the transcription factor SOX9, a protein capable of stimulating the dermal papilla of the hair follicle, counteracting the progressive miniaturisation of the same through a mechanism of action different from the previous and synergistic with it.

In order to better describe the present invention, some examples are given, which describe only some of the possible compositions of the invention. Although such examples use selected formulations in accordance with this invention, it is clear that such examples are illustrative only and not limiting.

All the components of the compositions of this invention are commercially available or can be easily prepared following procedures known in the art.

All parts, percentages and proportions referred to and indicated in the claims are understood to be the total weight of the composition, unless otherwise stated.

The names of the ingredients used and shown in the examples follow the International Nomenclature of Cosmetic Ingredients (INCI) and are those officially recognized in the cosmetic sector. The ingredient "Aqua" mentioned in the examples below is always deionised water.

### EXAMPLE 1: Two-phase hair loss prevention lotion for men (the two phases are divided into separate compartments and combined at the time of use by mixing them thoroughly) - % by weight

Phase A *(to be placed in the main compartment):* Aqua 59.70% Zinc Gluconate 0.90% Arginine 0.65% Gluconic Acid 1.20% Alcohol denat. 22.40% Serenoa Serrulata Fruit Extract 2.00% Propylene Glycol 1.50% Polysorbate-20 0.75% Menthol 0.15% Vanillyl Butyl Ether 0.05%
Part B *(to be placed in the secondary compartment)* Ascorbic Acid 10.00% Magnesium Stearate 0.70%
   *Preparation:*
   I) prepare part A (liquid) by simple mixing of the ingredients under constant stirring
   (II) prepare Part B (solid) by careful and thorough mixing of the two fine powder ingredients composing it
   (III) separately place part A in the main compartment (vial) and part B in the secondary compartment (cap-reservoir).

### EXAMPLE 2: Two-phase hair loss prevention lotion for women (the two phases are divided into separate compartments and combined at the time of use by mixing them thoroughly) - % by weight

Phase A *(to be placed in the main compartment):* Aqua 64.95% Zinc Gluconate 0.80% Arginine 0.55% Gluconic Acid 0.50% Gluconolactone 0.45% Alcohol denat. 21.20% Propylene Glycol 1.50% Polysorbate-20 0.75% Menthol 0.15% Polysorbate-80 0.15% Lecithin 0.15% Soy Isoflavones 0.10% Vanillyl Butyl Ether 0.05%
Part B *(to be placed in the secondary compartment)* Ascorbic Acid 8.00% Magnesium Stearate 0.70%
*Preparation:*
   I) prepare part A (liquid) by simple mixing of the ingredients under constant stirring
   (II) prepare Part B (solid) by careful and thorough mixing of the two fine powder ingredients composing it
   (III) separately place part A in the main compartment (vial) and part B in the secondary compartment (cap-reservoir).

### EXAMPLE 3: Two-phase scalp cream mask for men (the two phases are divided into separate compartments and combined at the time of use by mixing them thoroughly) - % by weight

Phase A *(to be placed in the main compartment):* Aqua 76.80% Zinc Gluconate 0.90% Arginine 0.75% Gluconic Acid 0.70% Gluconolactone 0.35% Serenoa Serrulata Fruit Extract 2.50% PEG-40 Castor Oil 1.60% Propylene Glycol 1.50% Polysorbate-20 0.90%
Part B *(to be placed in the secondary compartment)* Ascorbic Acid 8.75% Cocoglycerides 2.50% Isopropyl Myristate 2.50% Menthol 0.20% Vanillyl Butyl Ether 0.05%
*Preparation:*
   I) prepare part A (liquid) by simple mixing of the ingredients under constant stirring
   (II) prepare Part B (suspension of solids in liquid) by careful mixing of the ingredients which compose it
   (III) separately place part A in the main compartment (vial) and part B in the secondary compartment (cap-reservoir, tube or vial).

### EXAMPLE 4: Two-phase scalp cream mask for women (the two phases are divided into separate compartments and combined at the time of use by mixing them thoroughly) - % by weight

Phase A *(to be placed in the main compartment):* Aqua 81.40% Zinc Gluconate 0.80% Arginine 0.65% Gluconic Acid 0.50% Gluconolactone 0.45% PEG-40 Castor Oil 1.50% Propylene Glycol 1.50% Menthol 0.15% Polysorbate-80 0.15%
Part B *(to be placed in the secondary compartment)* Ascorbic Acid 7.75% Cocoglycerides 2.50% Isopropyl Myristate 2.50% Genistein 0.10% Vanillyl Butyl Ether 0.05%.
*Preparation:*
   I) prepare part A (liquid) by simple mixing of the ingredients under constant stirring
   (II) prepare Part B (suspension of solids in liquid) by careful mixing of the ingredients which compose it
   (III) separately place part A in the main compartment (vial) and part B in the secondary compartment (cap-reservoir, tube or vial).

### EXAMPLE 5: unisex, two-phase hair loss prevention lotion (the two phases are divided into separate compartments and combined at the time of use by mixing them thoroughly) - % by weight

Phase A *(to be placed in the main compartment):* Aqua 13.00% Zinc Gluconate 0.20% Arginine 0.15% Gluconolactone 0.15% Alcohol denat. 30.00% Propylene Glycol 50%
Part B *(to be placed in the secondary compartment)* Ascorbic Acid 3.00% Minoxidil 3.00% Silica 0.25% Magnesium Stearate 0.25%
*Preparation:*
   I) prepare part A (liquid) by simple mixing of the ingredients under constant stirring
   (II) prepare Part B (solid) by careful and thorough mixing of the four fine powder ingredients composing it
   (III) separately place part A in the main compartment (vial) and part B in the secondary compartment (cap-reservoir).

It is understood, however, that the invention is not to be considered limited to the particular arrangement illustrated above, which is merely an embodiment provided by way of example, but that various variations are possible, all within the reach of a person skilled in the art, while remaining within the scope of protection of the invention itself, as defined by the following claims.

## Claims

1. Formulation against androgenetic alopecia **characterized in that** said formulation comprises zinc and arginine gluconates combined with ascorbic acid, this last one acting as initiator.

2. The formulation according to claim 1), **characterized in that** the formulation further comprises gluconic acid or gluconolactone.

3. The formulation against androgenetic alopecia according to claim 1), **characterized in that** the formulation comprising zinc and arginine gluconates combined with ascorbic acid primarily acts through the inhibition of the 5-alpha reductase type II enzyme and secondarily through the induction of the SOX9 protein.

4. The formulation according to claim 1) wherein the arginine gluconate, essential component of the formulation of claim 1, is obtained during production starting from arginine base and salifying it with stoichiometric amounts of gluconic acid or of the relative precursor, the gluconolactone.

5. The formulation according to claim 1), **characterized in that** said formulation comprises the further addition of arginine in the form of free base, not salified.

6. The formulation according to claim 1), **characterized in that** said formulation comprises other ingredients such as excipients, preservatives, stabilizers, solvents, charges, fillers and the like, as well as adjuvants or synergistic compounds chosen from the group comprising Soy Isoflavones, Genistein, Serenoa Serrulata Fruit Extract, Minoxidil.

7. The formulation according to claim 1), **characterized in that** said formulation consists of two separate phases, to be mixed at the time of use or at a time very close to such use, to ensure the stability of the components over time and therefore the respective effectiveness.

8. The formulation according to claim 7), **characterized in that** said first phase of the formulation is liquid or creamy or in the form of a gel and contains zinc and arginine gluconates and adjuvants and excipients, and said second phase is in form of powder or suspension of solid particles in an oily or anhydrous vehicle and contains ascorbic acid and adjuvants and excipients.

9. The formulation according to claim 7), **characterized in that** the final preparation resulting from the union of said two phases is used for topical applications on the scalp according to methods of different types such as massages, brushing, sub-cutaneous injections, sprays, shampoos.

10. Kit formulation as defined in claim 7) consisting of two separate phases or parts, packaged in two different compartments, main and secondary compartments, to be mixed just before use.

11. The formulation according to claim 8), **characterized in that** said first phase, to be partitioned in said main compartment, contains, in percentage by weight: water from 50.00 to 90.00%, zinc gluconate from 0.10 to 6.00%, arginine from 0.20 to 12.00%, gluconic acid or gluconolactone from 0.20 to 12.00%, and **in that** said second phase, to be partitioned in said secondary compartment, contains ascorbic acid from 0.50 to 15.00%; with the clarification that the aforementioned percentages are to be understood as calculated with respect to the total of the 2 phases.

12. Method of preparation of the formulation according to claims 7) and 8), **characterized in that** the first liquid phase is prepared by simple mixing of the ingredients under constant stirring; the second phase - which is solid or in suspension of solid in liquid - is prepared through careful and prolonged mixing of the ingredients that compose it; the method further **characterized in that** the first phase in the main vial compartment and the second phase in the secondary tank cap or tube or vial compartment are separately partitioned.

## Patentansprüche

1. Formulierung gegen androgenetische Alopezie, **dadurch gekennzeichnet, dass** die Formulierung Zink- und Arginin-Gluconate in Kombination mit Ascorbinsäure umfasst, wobei letztere als Initiator wirkt.

2. Formulierung nach Anspruch 1), **dadurch gekennzeichnet, dass** die Formulierung ferner Gluconsäure oder Gluconolacton umfasst.

3. Formulierung gegen androgenetische Alopezie nach Anspruch 1), **dadurch gekennzeichnet, dass** die Formulierung, die Zink- und Arginin-Gluconate in Kombination mit Ascorbinsäure umfasst, primär durch die Hemmung des Enzyms 5-Alpha-Reduktase Typ II und sekundär durch die Induktion des SOX9-Proteins wirkt.

4. Formulierung nach Anspruch 1), wobei das Arginin-Gluconat, das ein wesentlicher Bestandteil der Formulierung nach Anspruch 1 ist, bei der Herstellung ausgehend von einer Argininbase erhalten wird, und diese mit stöchiometrischen Mengen an Gluconsäure oder dem entsprechenden Vorläufer, dem Gluconolacton, in ein Salz überführt wird.

5. Formulierung nach Anspruch 1), **dadurch gekennzeichnet, dass** die Formulierung den weiteren Zusatz von Arginin in Form einer freien Base, nicht in Salzform, umfasst.

6. Formulierung nach Anspruch 1), **dadurch gekennzeichnet, dass** die Formulierung andere Inhaltsstoffe wie Hilfsstoffe, Konservierungsmittel, Stabilisatoren, Lösungsmittel, Füller, Füllstoffe und dergleichen sowie Adjuvantien oder synergistische Verbindungen umfasst, die aus der Gruppe ausgewählt sind, die Soja-Isoflavone, Genistein, Serenoa-serrulata-Fruchtextrakt und Minoxidil umfasst.

7. Formulierung nach Anspruch 1), **dadurch gekennzeichnet, dass** die Formulierung aus zwei getrennten Phasen besteht, die zum Zeitpunkt der Verwendung oder zu einem Zeitpunkt kurz vor der Verwendung zu mischen sind, um die Stabilität der Bestandteile im Laufe der Zeit und damit die jeweilige Wirksamkeit zu gewährleisten.

8. Formulierung nach Anspruch 7), **dadurch gekennzeichnet, dass** die erste Phase der Formulierung flüssig oder cremeartig oder in Form eines Gels vorliegt und Zink- und Arginin-Gluconate sowie Adjuvantien und Hilfsstoffe enthält, und dass die zweite Phase in Form eines Pulvers oder einer Suspension von festen Partikeln in einem öligen oder wasserfreien Vehikel vorliegt und Ascorbinsäure sowie Adjuvantien und Hilfsstoffe enthält.

9. Formulierung nach Anspruch 7), **dadurch gekennzeichnet, dass** das Endpräparat, das aus der Vereinigung der beiden Phasen resultiert, für topische Anwendungen auf der Kopfhaut nach Verfahren verschiedener Art wie Massagen, Bürsten, subkutane Injektionen, Sprays, Shampoos verwendet wird.

10. Kit-Formulierung wie in Anspruch 7) definiert, die aus zwei getrennten Phasen oder Teilen besteht, die in zwei verschiedenen Fächern, einem Haupt- und einem sekundären Fach, die unmittelbar vor der Verwendung zu mischen sind, verpackt sind.

11. Formulierung nach Anspruch 8), **dadurch gekennzeichnet, dass** die erste Phase, die in das Hauptfach aufzuteilen ist, in Gewichtsprozent enthält: Wasser von 50,00 bis 90,00 %, Zink-Gluconat von 0,10 bis 6,00 %, Arginin von 0,20 bis 12,00 %, Gluconsäure oder Gluconolacton von 0,20 bis 12,00 %, und dadurch, dass die zweite Phase, die in das sekundäre Fach aufzuteilen ist, 0,50 bis 15,00 % Ascorbinsäure enthält; mit der Klarstellung, dass die vorgenannten Prozentsätze so zu verstehen sind, dass sie in Bezug auf die Summe der 2 Phasen berechnet werden.

12. Verfahren zur Herstellung der Formulierung nach den Ansprüchen 7) und 8), **dadurch gekennzeichnet, dass** die erste flüssige Phase durch einfaches Mischen der Inhaltsstoffe unter ständigem Rühren hergestellt wird; die zweite Phase - die fest oder in einer Suspension von Feststoffen in Flüssigkeit vorliegt - durch sorgfältiges und längeres Mischen der Inhaltsstoffe, aus denen sie besteht, hergestellt wird; wobei das Verfahren ferner **dadurch gekennzeichnet ist, dass** die erste Phase in dem Hauptfach des Fläschchens und die zweite Phase in dem sekundären Behälterverschluss oder Röhrchen oder Fach des Fläschchens getrennt aufgeteilt sind.

## Revendications

1. Formulation contre l'alopécie androgénétique **caractérisée en ce que** ladite formulation comprend des gluconates de zinc et d'arginine associés à de l'acide ascorbique, ce dernier agissant comme initiateur.

2. Formulation selon la revendication 1), **caractérisée en ce que** la formulation comprend en outre de l'acide gluconique ou de la gluconolactone.

3. Formulation contre l'alopécie androgénétique selon la revendication 1), **caractérisée en ce que** la formulation comprenant des gluconates de zinc et d'arginine combinés à de l'acide ascorbique agit principalement par l'inhibition de l'enzyme 5-alpha réductase de type II et secondairement par l'induction de la protéine SOX9.

4. Formulation selon la revendication 1) dans laquelle le gluconate d'arginine, composant essentiel de la formulation de la revendication 1, est obtenu lors de la production à partir de l'arginine base et en la salifiant avec des quantités stœchiométriques d'acide gluconique ou du précurseur relatif, la gluconolactone.

5. Formulation selon la revendication 1), **caractérisée en ce que** ladite formulation comprend l'addition supplémentaire d'arginine sous forme de base libre, non salifiée.

6. Formulation selon la revendication 1), **caractérisée en ce que** ladite formulation comprend d'autres ingrédients tels que des excipients, des conservateurs, des stabilisants, des solvants, des charges, des remplisseurs et similaires, ainsi que des adjuvants ou des composés synergiques choisis dans le groupe comprenant les Isoflavones de Soja, la Génistéine, l'Extrait de Fruit Serenoa Serrulata, le Minoxidil.

7. Formulation selon la revendication 1), **caractérisée en ce que** ladite formulation est constituée de deux phases distinctes, à mélanger au moment de l'utilisation ou à un moment très proche de cette utilisation, pour assurer la stabilité des composants dans le temps et donc l'efficacité respective.

8. Formulation selon la revendication 7), **caractérisée en ce que** ladite première phase de la formulation est liquide ou crémeuse ou sous forme de gel et contient des gluconates de zinc et d'arginine et des adjuvants et excipients, et ladite deuxième phase est sous forme de poudre ou de suspension de particules solides dans un véhicule huileux ou anhydre et contient de l'acide ascorbique et des adjuvants et excipients.

9. Formulation selon la revendication 7), **caractérisée en ce que** la préparation finale résultant de l'union desdites deux phases est utilisée pour des applications topiques sur le cuir chevelu selon des méthodes de types différents telles que les massages, le brossage, les injections sous-cutanées, les sprays, les shampooings.

10. Formulation de kit telle que définie dans la revendication 7) consistant en deux phases ou parties séparées, emballées dans deux compartiments différents, les compartiments principal et secondaire, à mélanger juste avant l'utilisation.

11. Formulation selon la revendication 8), **caractérisée en ce que** ladite première phase, à répartir dans ledit compartiment principal, contient, en pourcentage en poids : de l'eau de 50,00 à 90,00 %, du gluconate de zinc de 0,10 à 6,00 %, de l'arginine de 0,20 à 12,00 %, de l'acide gluconique ou de la gluconolactone de 0,20 à 12,00 %, et **en ce que** ladite deuxième phase, à répartir dans ledit compartiment secondaire, contient de l'acide ascorbique de 0,50 à 15,00 % ; avec la précision que les pourcentages susmentionnés doivent être compris comme calculés par rapport au total des 2 phases.

12. Procédé de préparation de la formulation selon les revendications 7) et 8), **caractérisé en ce que** la première phase liquide est préparée par simple mélange des ingrédients sous agitation constante ; la deuxième phase, qui est solide ou en suspension de solide dans le liquide, est préparée par mélange soigneux et prolongé des ingrédients qui la composent ; le procédé étant en outre **caractérisé en ce que** la première phase dans le compartiment principal du flacon et la deuxième phase dans le compartiment secondaire du bouchon de réservoir ou du tube ou du flacon sont cloisonnées séparément.
